# EUROPEAN PATENT APPLICATION

(11) **EP 1 774 933 A1**
(43) Date of publication of application: **18.04.2007**
(21) Application number: 05022569.7
(22) Date of filing: 17.10.2005
(51) Int. Cl.: A61F 5/455

(54) **Urination aid**

(71) Applicant: Carella, Pietro, 10045 Piossasco, TO (IT)
(72) Inventor: Carella, Pietro, 10045 Piossasco, TO (IT); Cammarota, Michele, 10045 Piossasco, TO (IT)

(57) **Abstract**

The application concerns an article for intimate hygiene, in particular an urination aid.

## Description

This patent concern an article for intimate hygiene, directly connected with female needs,

Liber@ female ejector is helpful on many occasions and occurrences such as:
- Far from home
- During trip
- In hospital
- For disable person
- When necessary away from toilet

The main function of Liber@ ant its particular benefit has been thought and then created to grant an high hygienic level to people with motor deficiency and difficulties.

To let more comfortable the use of Liber@ you will find in every box:
- A glove and a small towel to grant the highest hygiene performance,
   After use, the throw-away glove can be utilized as an envelope
   Liber@ could be made in biodegradable and/or recycling material, made in single closed envelope and ready in boxes of one ore more envelopes,
   Liber@ is a symbol of freedom and meets the natural needs of a daily women life

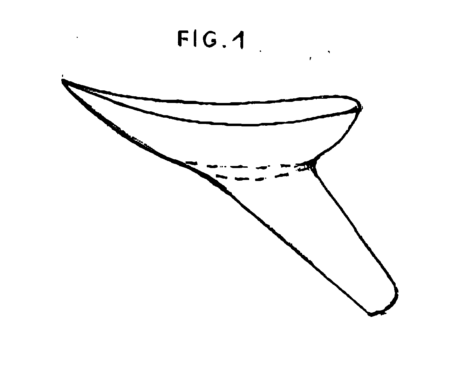

## Claims

**1.** This patent concern an article for intimate hygiene, directly connected with female needs.

**2.** Liber@ female ejector is helpful on many occasions and occurrences such as:
• Far from home
• During trip
• In hospital
• For disable person
• When necessary away from toilet

**3.** The main function of Liber@ ant its particular benefit has been thought and then created to grant an high hygienic level to people with motor deficiency and difficulties.

**4.** To let more comfortable the use of Liber@ you will find in every box;
• A glove and a small towel to grant the highest hygiene performance,
After use, the throw-away glove can be utilized as an envelope
Liber@ could be made in biodegradable and/or recycling material, made in single closed envelope and ready in boxes of one ore more envelopes,
Liber@ is a symbol of freedom and meets the natural needs of a daily women life
